# EUROPÄISCHE PATENTSCHRIFT

(11) **EP 0 681 822 B1**
(45) Veröffentlichungstag und Bekanntmachung des Hinweises auf die Patenterteilung: **01.12.1999**
(21) Anmeldenummer: 95105594.6
(22) Anmeldetag: 13.04.1995
(51) Int. Cl.: A61G 12/00, A61G 13/00, H02G 11/00

(54) **Versorgungseinheit für medizinische Behandlungsgeräte**
Supply unit for apparatus for medical treatment
Unité d'alimentation pour appareils pour traitement médical

(30) Priorität: 11.05.1994 DE 4416618
(43) Veröffentlichungstag der Anmeldung: 15.11.1995
(73) Patentinhaber: DRÄGERWERK AKTIENGESELLSCHAFT, 23542 Lübeck (DE)
(72) Erfinder: Kummerfeld, Ryszard, D-23570 Travemünde (DE)

(56) Entgegenhaltungen:
- EP-A- 0 215 212
- DE-U- 8 304 407
- DE-U- 8 706 822

## Beschreibung

Die Erfindung betrifft eine Versorgungseinheit für medizinische Behandlungsgeräte nach dem Oberbegriff des Patentanspruchs 1.

Aus dem Gebrauchsmuster DE-U 87 06 822 ist eine Versorgungseinheit für ein medizinisches Behandlungsgerät bekannt, bei dem Versorgungsleitungen innerhalb eines Versorgungsbalkens schlaufenförmig aufgenommen und über eine Schlauchführung aus dem Versorgungsbalken heraus zu einem am Versorgungsbalken längsverschiebbar angeordneten Wagen mit einer Tragevorrichtung umgelenkt sind. Die Teillängen der frei verschiebbaren Versorgungsleitungsabschnitte innerhalb des Versorgungsbalkens sind derart bemessen, daß sie einem vorbestimmten Bewegungshub des Wagens entsprechen.

Aus der EP-A 215 212 ist eine Versorgungseinheit bekannt, die aus einem an einer Decke befestigten, hohlprofilartigen Versorgungsbalken besteht, an dessen Unterseite eine am Versorgungsbalken längsverlaufende Führungsbahn angebracht ist. Die Führungsbahn besteht aus zwei parallelen Schienen, an denen ein Schlitten mit einer Tragevorrichtung für medizinische Behandlungsgeräte längsverschiebbar geführt wird. Durch Verschieben des Schlittens längs der Schienen, kann die Tragevorrichtung in eine günstige Position, z.B. zu einem Patientenbett, gebracht werden. An den zur Decke abgewinkelten, längsverlaufenden Seitenteilen des Versorgungsbalkens sind Versorgungsanschlüsse, wie z.B. Steckdosen und gasmedizinische Kupplungen angebracht, die zur Energieversorgung von den auf der Tragevorrichtung befindlichen Behandlungsgeräten dienen. Hierzu werden die aus den Behandlungsgeräten kommenden Energieversorgungsleitungen in die Versorgungsanschlüsse am Versorgungsbalken eingesteckt. Nachteilig bei der bekannten Versorgungseinheit ist, daß das Verschieben des Schlittens auf der Führungsbahn durch zu kurze Energieversorgungsleitungen behindert sein kann und im Bedarfsfall dann diese Energieversorgungsleitungen durch längere ausgetauscht werden müssen. Dieses erschwert die Handhabung im klinischen Routinebetrieb. Auf der anderen Seite sind zu lange Energieversorgungsleitungen auch nicht erwünscht, da hierdurch die Leitungsanordnung unübersichtlich wird.

Aus dem DE-GM 83 04 407 ist eine Tragevorrichtung für medizinische Geräte bekanntgeworden, welche mittels eines an einer Führungsbahn längsverschiebbaren Wagens positionierbar ist. Mittels eines an der Führungsbahn befestigten, ebenfalls verschiebbaren Kabelwagens werden elektrische Versorgungsleitungen zu Versorgungsanschlüssen an der Tragevorrichtung geführt, so daß die Energieversorgungsleitungen der medizinischen Geräte unmittelbar an Versorgungsanschlüsse an der Tragevorrichtung angeschlossen werden können.

Nachteilig bei der bekannten Vorrichtung ist, daß die freitragende Leitungsführung über den Kabelwagen aus Reinigungsgründen und wegen der Optik abgelehnt wird und auch nur für leichte Versorgungsleitungen, wie z.B. Elektrokabel, geeignet ist, nicht aber für ein Bündel von gasmedizinischen Schläuchen.

Der Erfindung liegt die Aufgabe zugrunde, eine Versorgungseinheit der eingangs genannten Art derart zu verbessern, daß die Versorgungsleitungen weitgehend verdeckt geführt sind, ohne daß die Verschiebbarkeit der Tragevorrichtung längs des Versorgungsbalkens eingeschränkt wird.

Die Lösung der Aufgabe erfolgt mit den Merkmalen des Patentanspruchs 1.

Der Vorteil der Erfindung besteht im wesentlichen darin, daß die Schlauchführung als ein mit dem Wagen oder mit der Tragevorrichtung verbundenes Umlenkblech ausgeführt ist, welches längs einer Profilrückseite des Versorgungsbalkens verläuft. Die Profilvorderseite und die Profilrückseite sind winklig zur Unterseite des Versorgungsbalkens abgekantet und verlaufen längs der Führungsbahnen, an denen der Wagen mit der Tragevorrichtung verschiebbar ist. In zweckmäßiger Weise ist das Umlenkblech zumindestens bis an die Oberkante der Profilrückseite geführt, um eine gute Umlenkung der Versorgungsleitungen aus dem Innenraum des Versorgungsbalkens heraus zu der Tragevorrichtung zu gewährleisten. Das Umlenkblech kann auch über die Oberkante der Profilrückseite in den Innenraum des Versorgungsbalkens hinein abgekantet sein, um die Versorgungsleitungen vor Beschädigungen zu schützen.

Das Umlenkblech ist an der für den Benutzer nicht sichtbaren Profilrückseite angeordnet und die Höhe h₁ der Profilvorderseite und die Höhe h₂ der Profilrückseite sind derart ausgelegt, daß h₂ kleiner als h₁ ist. Wird die Höhe h₂ derart bemessen, daß sie zumindestens um die Stärke der Versorgungsleitungen kleiner als h₁ ist, wird die Leitungsführung für einen Benutzer, dessen Blick gegen die Profilvorderseite gerichtet ist, vollständig verdeckt.

Die Versorgungsanschlüsse sind in unmittelbarer Nähe der auf der Tragevorrichtung befindlichen Behandlungsgeräte angeordnet, und es ist möglich, die Länge der einzelnen Energieversorgungsleitungen zwischen den Versorgungsanschlüssen und den Behandlungsgeräten auf ein Mindestmaß zu beschränken, wodurch die Übersichtlichkeit der Leitungsführung wesentlich erhöht wird.

Ein Ausführungsbeispiel der Erfindung ist in der Zeichnung dargestellt und im folgenden näher erläutert.

Es zeigen:
- Figur 1:: eine erste Ausführungsform einer Versorgungseinheit in perspektivischer Darstellung,
- Figur 2:: eine Seitenansicht der Versorgungseinheit nach der Figur 1 in Blickrichtung "A",
- Figur 3:: eine Schnittdarstellung der Versorgungsleitungen.

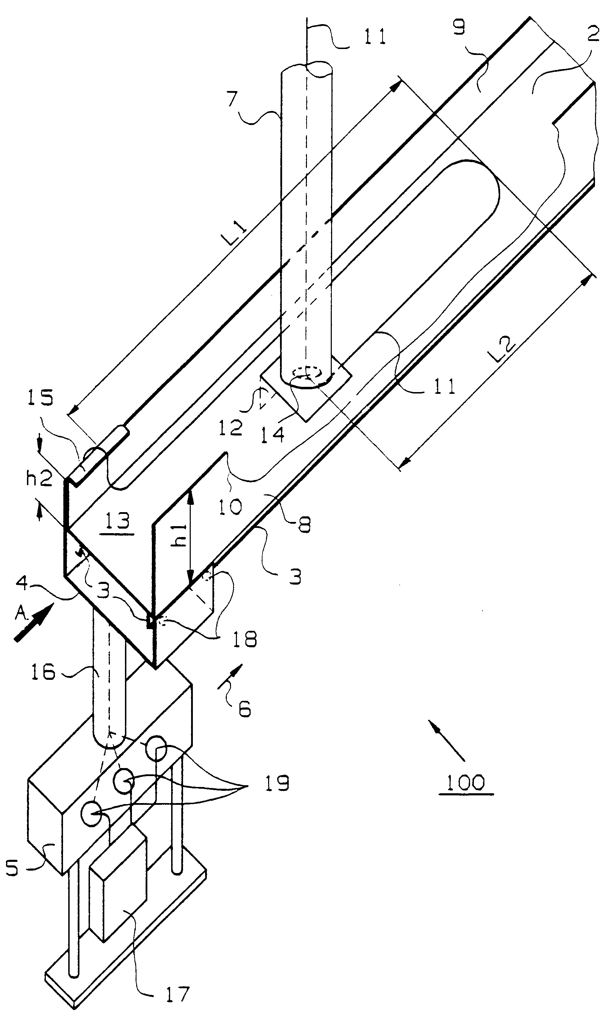

Figur 1 zeigt in perspektivischer Ansicht eine erste Versorgungseinheit (100), mit einem Versorgungsbalken (2) und mit Führungsbahnen (3) an der Unterseite (13) des Versorgungsbalkens (2), auf denen ein Wagen (4) mit einer Tragevorrichtung (5) längsverschiebbar geführt ist. Die Verschieberichtung ist durch einen Pfeil (6) veranschaulicht. In der in der Figur 1 gezeigten Position befindet sich der Wagen (4) am linken Anschlag des Versorgungsbalkens (2). Der Versorgungsbalken (2) ist mittels Trageprofilen (7) an der Decke befestigt. Von den Trageprofilen (7) ist in der Figur 1 nur das vorderste Trageprofil (7) dargestellt. Der Versorgungsbalken (2) besteht aus einem U-förmigen Profil mit einer Profilvorderseite (8) der Höhe h₁ und einer Profilrückseite (9) der Höhe h₂. Der besseren Übersicht wegen ist die Profilvorderseite (8) längs einer Schnittlinie (10) aufgeschnitten. Das Trageprofil (7), welches hohlprofilartig zur Aufnahme von Versorgungsleitungen (11) ausgebildet ist, ist mittels eines Winkels (12) an Versorgungsbalken (2) befestigt, der an die Profilvorderseite (8) und die Unterseite (13) angeschweißt ist. Im Bereich der Verbindungsstelle zwischen dem Winkel (12) und dem Trageprofil (7) besitzt der Winkel (12) eine Bohrung (14), durch die die Versorgungsleitungen (11) in das wannenartige Profil des Versorgungsbalkens (2) eingeführt werden können. Die Versorgungsleitungen (11) sind in der Figur 1 nur schematisch durch eine Strichlinie veranschaulicht; sie bestehen normalerweise aus einem Bündel von gasmedizinischen und elektrischen Leitungen. Die Versorgungsleitungen (11) liegen auf der Unterseite (13) des Versorgungsbalkens schlaufenförmig auf und werden mittels eines Umlenkbleches (15) als Schlauchführung, welches mit dem Wagen (4) fest verbunden ist und parallel zur Profilrückseite (9) verläuft, in Richtung der Tragevorrichtung (5) umgelenkt. Die Tragevorrichtung ist mittels eines Rohres (16) an dem Wagen (4) befestigt. An der Tragevorrichtung (5) sind Versorgungsanschlüsse (19) angebracht, die mit den Versorgungsleitungen (11) verbunden sind und zur Energieversorgung eines auf der Tragevorrichtung (5) befindlichen medizinischen Behandlungsgerätes (17) dienen. Der Wagen ist mittels Rollen (18) auf den Führungsbahnen (13) verschiebbar.

Wird nun der Wagen (4) längs des Pfeils (6) verschoben, verändern sich die Teillängen L₁ und L₂ der frei verschiebbaren Versorgungsleitungen (11) innerhalb des Versorgungsbalkens (2); d.h. L₂ nimmt in dem Maße zu, wie L₁ abnimmt. Hierdurch ist es möglich, die Versorgungsleitungen (11) immer der momentanen Position des Wagens (4) nachzuführen. Die Gesamtlänge L₁ + L₂ der Versorgungsleitungen (11) innerhalb des Versorgungsbalkens (2) ergibt sich aus dem Bewegungshub, den der Wagen (4) auszuführen hat. Um die Leitungsführung längs des Umlenkbleches (15) für einen vor dem Behandlungsgerät (17) stehenden Benutzer mit Blick auf die Profilvorderseite (8) verdeckt zu halten, ist das Profil des Versorgungsbalkens (2) derart ausgeführt, daß die Höhe h₁ der Profilvorderseite (8) größer als die Höhe h₂ der Profilrückseite (9) ist.

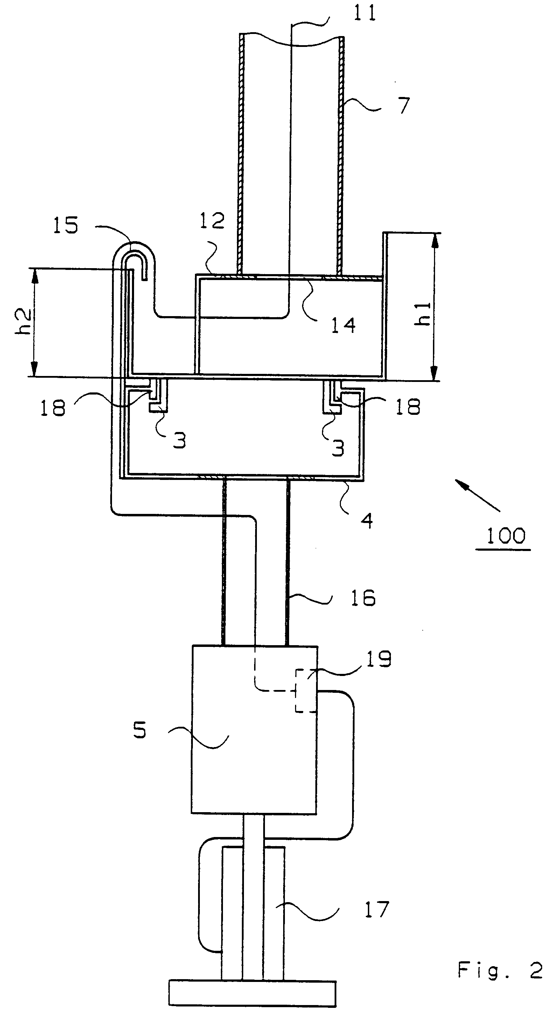

Figur 2 zeigt eine Seitenansicht der ersten Versorgungseinheit (100) in Blickrichtung "A" nach der Figur 1. Gleiche Komponenten sind mit gleichen Bezugsziffern der Figur 1 bezeichnet. Der besseren Übersicht wegen sind die Versorgungsleitungen (11) mit einem Abstand zum Umlenkblech (15) gezeichnet.

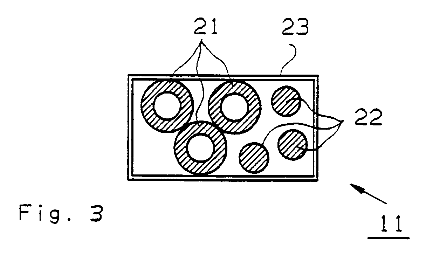

Figur 3 zeigt die Versorgungsleitungen (11) im Längsschnitt, welche aus gasmedizinischen Leitungen (21) und elektrischen Leitungen (22) bestehen und mit einer schlauchförmigen Ummantelung (23) zusammengehalten werden.

## Patentansprüche

1. Versorgungseinheit für medizinische Behandlungsgeräte mit einem an einer Decke befestigten, hohlprofilartigen Versorgungsbalken (2) mit einer Profilvorderseite (8) und einer Profilrückseite (9), welcher elektrische und gasmedizinische Versorgungsleitungen enthält, mit am Versorgungsbalken (2) längsverlaufenden Führungsbahnen (3), auf denen ein Wagen (4) mit einer Tragevorrichtung (5) längsverschiebbar geführt ist, mit innerhalb des Versorgungsbalkens (2) schlaufenförmig, mit Teillängen L₁ und L₂ aufgenommenen Versorgungsleitungen (11), die über eine Schlauchführung aus dem Versorgungsbalken (2) heraus zu der Tragevorrichtung (5) umgelenkt sind, wobei die Teillängen L₁ und L₂ derart bemessen sind, daß sie einem vorbestimmten Bewegungshub des Wagens (4) entsprechen, dadurch gekennzeichnet, daß die Schlauchführung ein mit dem Wagen (4) verbundenes Umlenkblech (15) ist, welches an der für einen Benutzer nicht sichtbaren Profilrückseite (9) des Versorgungsbalkens (2) angeordnet ist, und daß das Profil des Versorgungsbalkens (2) derart ausgeführt ist, daß die Höhe h₂ der Profilrückseite (9) kleiner als die Höhe h₁ der Profilvorderseite (8) ist.

## Claims

1. Supply unit for medical treatment devises having a highly profiled-type supply beam (2) secured to a cover and having a profiled front side (8) and a profiled rear side (9), which unit contains electrical and medical-gas supply lines, having guideways (3) which extend longitudinally on the supply beam (2) and on which a carriage (4) with a carrier device (5) is guided longitudinally displaceably, having supply lines (11) accommodated as loops with part lengths L₁ and L₂ inside the supply beam (2) which are guided by means of a hose guide means from the supply beam (2) to the carrier device (5), the part lengths L₁ and L₂ are dimensioned in such a way that they correspond to a specific movement extent of the carriage (4), characterised in that the hose guide means is a baffle (15) connected to the carriage (4), this baffle being arranged on the profiled rear side (9) of the supply beam (2) not visible to the user, and in that the profile of the supply bar (2) is designed so that the height h₂ of the profiled rear side (9) is less than the height h₁ of the profiled front side (8).

## Revendications

1. Unité d'alimentation pour appareils de traitements médicaux, comprenant une poutre d'alimentation (2), du type en profilé creux, fixée sur un plafond, présentant une face avant profilée (8) et une face arrière profilée (9), qui renferme des conduites d'alimentation électrique et d'alimentation en gaz à usage médical, comprenant des glissières de guidage (3), s'étendant longitudinalement sur la poutre d'alimentation (2), sur lesquelles un chariot (4) équipé d'un dispositif porteur (5) est guidé avec possibilité de déplacement longitudinal, comprenant des conduites d'alimentation (11) en forme de boucles, logées, par des longueurs partielles L₁ et L₂, à l'intérieur de la poutre d'alimentation (2), qui, par l'intermédiaire d'un guide-tuyau, sont amenées à sortir de la poutre d'alimentation (2) pour s'étendre en direction du dispositif porteur (5), les longueurs partielles L₁ et L₂ présentant des dimensions telles qu'elles correspondent à une course prédéterminée du chariot (4), caractérisée en ce que le guide-tuyau est une tôle de dérivation (15) reliée au chariot (4), placée sur une face arrière profilée (9), non visible par un utilisateur, de la poutre d'alimentation (2), et en ce que le profil de la poutre d'alimentation (2) est conçu de telle sorte que la hauteur h₂ de la face arrière profilée (9) est inférieure à la hauteur h₁ de la face avant profilée (8).
